Europäisches Patentamt

European Patent Office

Office européen des brevets

(11) Veröffentlichungsnummer: **0 279 392**
**A2**

(12) **EUROPÄISCHE PATENTANMELDUNG**

(21) Anmeldenummer: **88102152.1**

(22) Anmeldetag: **13.02.88**

(51) Int. Cl.⁴: **A61F 7/00**

(30) Priorität: **20.02.87 CH 648/87**

(43) Veröffentlichungstag der Anmeldung:
**24.08.88 Patentblatt 88/34**

(84) Benannte Vertragsstaaten:
**AT BE CH DE ES FR GB GR IT LI LU NL SE**

(71) Anmelder: **MODIMA SA**
**Hinterbergstrasse 28**
**CH-6330 Cham(CH)**

(72) Erfinder: **Beck, Julius, H.**
**Winkelstrasse 4**
**6048 Horw(CH)**

(74) Vertreter: **EGLI-EUROPEAN PATENT**
**ATTORNEYS**
**Horneggstrasse 4**
**CH-8008 Zürich(CH)**

(54) **Kosmetisches Präparat.**

㊿ Ein Gehalt an nach Champagnerart hergestelltem Schaumwein, insbesondere Champagner, steigert die Wirksamkeit von kosmetischen Präparaten unerwartet. Dabei wird vorzugsweise der überweigende Teil der wässrigen Phase solcher Präparate aus flüssigen Bestandteilen solchen Schaumweins, insbesondere Champagners, bestehen.

EP 0 279 392 A2

## Kosmetisches Präparat

Die Erfindung betrifft ein kosmetisches Präparat nach dem Obergriff des Anspruch 1.

Die in derartigen kosmetischen Präparaten enthaltene wässrige Phase, die als Träger mannigfacher Substanzen dient, besteht herkömmlicherweise aus Wasser. man ist bei der Herstellung solcher Präparate selbstverständlich bestrebt, alles zu vermeiden, was zu Störungen im Präparat oder bei seiner späteren Verwendung führen könnte. Daher gilt es als unumstössliche Regel, nur reinstes Wasser, also durch Destillation oder auf anderem Wege demineralisiertes Wasser zu verwenden.

Ferner beschreibt die CH-PS Nr. 563 162 (1975) ein kosmetisches Produkt, das vorwiegend auf der Basis von Ethylalkohol hergestellt ist.

In der EP-AS Nr. 196 340 (1986) ist ein kosmetisches Produkt auf der Basis von einer Mischung aus Ethanol und Traubenwein beschrieben.

Zudem beschreibt die DE-PS Nr. 36 05 570 (1986) ein Mittel zum straffen von menschlichen Hautpartien auf der Basis von Rum.

Schliesslich beschreibt die CH-PS Nr. 659 943 (1987) ein antiseptisches Produkt auf der Basis von Wein und Honig. Diese Patentschrift ·wurde allerdings erst am 13. März 1987, also nach dem Prioritätsdatum 20. Februar 1987 des vorliegenden Gesuches veröffentlicht und gilt somit nicht als Stand der Technik.

Der Erfindung liegt die Aufgabe zugrunde, kosmetische Präparate mit gegenüber herkömmlichen Ausführungen verbesserten Eigenschaften zu schaffen.

Bei mit dieser Zielsetzung durchgeführten umfangreichen Versuchen ergab sich überraschenderweise, dass man Präparate mit unerwartet gesteigerter Wirksamkeit erhält, wenn diese einen nach Champagnerart hergestellten Schaumwein, insbesondere Champagner enthalten.

Dementsprechend wird die Erfindungsaufgabe durch die im Kennzeichnungsteil des Anspruches 1 definierten kosmetischen Präparate gelöst.

Ganz besonders gute Resulte erhält man, wenn die (sonst aus demineralisiertem Wasser bestehende) wässrige Phase wenigstens grösstenteils durch flüssige Bestandteile eines genannten Schaumweins, insbesondere Champagner gebildet ist.

Aus der Rebsorte Pinot-Noir gewonnener Schaumwein der genannten Art, insbesondere Champagner ergibt in kosmetischen Präparaten besonders überraschende Aktivitäten.

Weisser Schaumwein der genannten Art, insbesondere Champagner erweist sich nicht nur als relativ farbneutral, sonderen erlaubt es, seine Eigenschaften besonders günstig zum Ausdruck kommen zu lassen, wobei ein Blanc-de-Noir, besonders, wenn er aus Pinot-Noir-Trauben gewonnen wurde, sich hervorragend für die Zwecke der Erfindung eignet.

Aber auch aus weissen Trauben gewonnener weisser Schaumwein der genannten Art, wiederum vorzugsweise Champagner, eignet sich in viele Fällen hervorragend.

Rosé-Schaumwein bzw. Champagner kann dort mit Erfolg eingesetzt werden, wo seine färbende Wirkung nicht störend empfunden wird, was naturgemäss im vermehrten Masse für roten Schaumwein bzw. Champagner gilt.

Anfänglich tappte man hinsichtlich der Ursachen der überraschenden Effekte im Dunkeln. Wenn auch mit einiger Spekulation belastet, glaubt man nun die Ursachen der überraschenden Effekte des Schaumweins der genannten Art und insbesondere des Champagners in den erfindungsgemässen kosmetischen Präparaten deuten zu können. Die diesbezüglich nachstehend gemachten Angaben werden aber unter dem ausdrücklichen Vorbehalt gemacht, das durch darin enthaltene Fehlspekulationen die Erfindung als solche nicht beeinflusst werden soll.

Eine durch den Erfinder durchgeführte Analyse eines Champagners hat neben Wasser folgende Bestandteile ergeben:

Mineralstoffe: Verbindungen von Ca, K, Na, Mg, Mn, FE, B, Si, Cl, I und CU (insgesamt ca. 3 Gew.-%).

Stickstoffverbindungen: Albumine, Proteine, Xanthin, Sarkin, Cholin, Prolin, Lysin, Histidin, Arginin, Cystin und Lecitin.

Kohlehydrate: Dextrose, Pentose, Stärkezucker, Lävulose und Glucose.

Natürliche Säuren: Weinsäure, Apfelsäure, Milchsäure, Kieselsäure, Benzoesäure, Zitronensäure, Salicylsäure und flüchtige Säuren (insgesamt ca. 5 bis 9 Gew.%).

Vitamine: A, B1, B2, B5, B6, C und D.

Alkohole: Aethylalkohol, Buthylenglycol, Sorbit und Mamit.

Natürliche Farbstoffe: Oenin, Xanthophyll und Carotin.

Der Champagner enthält eine Vielzahl hautfreundlicher Bestandteile in ausgewogener Kombination. Nachträglich ist man erstaunt, dass man nicht schon lange Champagner als Träger der darin enthaltenen und von anderen Stoffen eines kosmetischen Präparates verwendete und das leere demineralisierte Wasser wenigstens teilweise durch diesen Spender hautfreundlicher Stoffe in der wässrigen Phase ersetzt hat.

Die genannten, der Haut überraschend zuträglichen, natürlich entstandenen hautfreundlichen Eigenschaften des Champagners lassen sich ideal mit neuesten Renutritive-Repair-Komplexen (Zellenextrakt enthaltend) kombinieren, und ergeben Wirkungen, wie sie von der modernen Kosmetik anvisiert wurden:

- Die Zellbildug und Erneuerung wird beschleunigt.
- Die Fähigkeit der Haut, Feuchtigkeit zu speichern, wird erhöht und zugleich wird ihr vermehrt Feuchtigkeit zugeführt.
- Das biologische Gleichgewicht der Haut wird günstig beeinflusst.
- Dank der bakteriologischen Wirkung werden hautschädliche Keime beseitigt, die gesunde Hautflora jedoch erhalten.
- Gereizte Haut wird beruhigt und widerstandsfähiger.

Das hat zur Folge:
- Die Aktivierung der Zellbildung verlangsamt den Alterungsprozess, so dass die Haut länger jung und frisch bleibt.
- Durch die verbesserten Feuchtigkeitsbedingungen werden erschlaffte Zellen gefüllt, so dass die Haut elastisch und jugendlich bleibt.
- Die Zuführung der biologisch wertvollen Stoffe fördert die natürliche Funktionsfähigkeit der Haut, was auch schädlichen Umwelteinflüssen entgegenwirkt und die Widerstandskraft erhöht, so dass die Haut gesund bleiben kann. Gesunde Haut ist - schöner.
- Bakteriologisch fällt besonders ins Gewicht, dass der Champagner das Erhalten des natürlichen Säurepanzers der Haut unterstützt und gegen das Wachstum von Keimen, Pilzen, Bakterien wirkt, wobei auch Akne günstig beeinflussbar ist. Folglich ist die Haut fein und glatt.
- Selbst gereizte Haut kann durch Behandlung mit einem erfindungsgemässen Präparat schnell beruhigt werden. Entzündungen klingen ab, Microrisse trockener Haut werden vermieden oder heilen rasch. Eine porentiefe Reinigung ist unter Desinfizierung erzielbar.

All das fördert die Erhaltung und Bildung zarter, samtiger, weicher und jugendlicher gesunder Haut, was schliesslich das Ziel aller kosmetischen Bemühungen ist.

Ein erfindungsgemässes kosmetisches Präparat kann von der Haut sehr leicht aufgenommen werden, so dass die pflegenden Substanzen sich in der Haut gut verteilen. Eine auf den Champagner zurückzuführende prickelnde Frische kann als besonders angenehm gelten.

Ein erfindungsgemässes kosmetisches Präparat kann beispielsweise als Reinigungsmilch, Gesichtslotion, Tagescrème, Nachtcrème, Augenkonturencrème, Gesichtsmaske, Day-and-Night-Repair, Peeling-Crème, Body Lotion und dergleichen vorliegen.

Drei Beispiele erfindugsgemässer kosmetischer Präparate werden nachstehend samt ihrer Herstellung geschildert (GW bedeutet dabei Gewichtsteile):

Beispiel 1:

Man schmilzt und vermischt bei 75 Celsiusgraden

12.00 GT Oelsäurepolyglycerinester (Hostacerin von Hoechst)

2.00 GT Cetylstearylalkohol (Emulgade von Henkel)

3.00 GT Paraffinöl

4.00 GT Neutralöl (Miglyol)

3.00 PCL-liquid (Dragoco)

0.03 GT Buthylhydroxyanisol (Oxynex).

In 60.00 GT Champagner löst man

0.20 GT Emulgator (Euxyl K-100 von Schülke + Mayer GmbH, D-2000 Glashütte)

4.00 GT Propylenglycol

2.00 GT "Rindermilzextrakt (Revitalin-P von Pentapharm Ltd, CH-Basel)

3.00 GT Kohlehydrat (Pentavitin von Pentapharm Ltd, CH-Basel)
und erhitzt auf 75 Celsiusgrade, lässt

0.50 GT Acrylsäurepolymer (Carbopol von Goodrich) quellen und fügt

0.70 GT Trimethanolamin, gelöst in

4.00 Wasser hinzu.

Unter ständigem Rühren mischt man die champagnerhaltige Flüssigkeit zur vorgenannten Schmelze, kühlt auf ca. 55 Celsiusgrade, homogenisiert und kühlt auf ca. 30 Celsiusgrade ab, worauf man

0.10 GT Kathon CG (ein Konservierungsmittel) und

0.40 GT Parfum hinzufügt und kaltrührt.

Man erhält so eine vorzügliche Tagescrème mit den vorgenannten Eigenschaften.

Beispiel 2

Man erhitzt auf 75 Celsiusgrade, schmilzt und vermischt

3.0 GT Sorbitansequioleat

5.0 GT Petrol Lanolin Alkohol (Amerchol CAB von Amerchol, USA)

2.5 GT Isopropylmyristat

0.5 GT Dragosantol (von Dragoco)

20.0 GT Paraffinum durum (Lunacera PEP von Fuller, D)

0.05 GT Konservierungsmittel (Oxynex 2004 von Merck AG, D-Darmstadt).

In 53.95 GT Champagner

löst man und erhitzt auf 75 Celsiusgrad

0.1 GT Konservierungsmittel (Kanthon CG von Christ, CH-Basel)

0.2 GT Hydroxymethyl-dioxo-imidazolinyl (Germall 115, von Sutton Labs.)

0.3 GT MgSO$_4$·H$_2$O

4.0 GT Rindermilzextrakt (Revitalin-P von Pentapharm Ltd., CH-Basel)

5.0 GT Bindegewebe von Augen (Zellenextrakt) (Pentaglycan von Pentapharm Ltd., CH-Basel)

5.0 GT Sorbit 60 (Karion F von Merck AG, D-Darmstadt).

Unter ständigem Rühren gibt man die heisse champagnerhaltige Flüssigkeit zur erstgenannten heissen Schmelze, kühlt auf ca. 30 Celsiusgrad ab, fügt dann

0.4 GT Parfum hinzu, mischt gründlich und kühlt auf Raumtemperatur.

Man erhält eine Augenkonturencrème mit unerwartet hoher Aktivität.


Beispiel 3:

Man erhitzt auf 70 Celsiusgrade, schmilzt und mischt

6.0 GT Sorbitansesquioleat (Arlacel 83 von Atlas/ICI)

2.0 GT Polyoxyäthylen-sorbitantrioleat (Tween 85 von Atlas/ICI)

5.0 GT 2-Octyl.dodecanol

5.0 GT Paraffiniöl

4.0 GT Bienenwachs

4.0 GT Isopropylpylmitat

4.0 GT Neutralöl (Miglyol von Dynamit Nobel, D)

1.0 GT Aluminiumstearat

2.0 GT Lanolin

0.5 GT Konservierungsmittel (Phenonip von Nipa Labs. GB).

In 54.3 GT Champagner löst man und erhitzt auf 70 Celsiusgrad

4.0 GT Propylenglycol

2.0 GT Glycerin

0.1 GT Allantoin

0.3 GT Magnesiumsulfat

0.3 GT Hydroxymethyl-dioxo-imidazolinyl (Germall 115 von Sutton Labs. USA)

5.0 GT Rindermilzextrakt (Revitalin-P von Pentapharm Ltd., Ch-Basel).

Unter ständigem Umrühren fügt man die heisse champagnerhaltige Flüssigkeit in die erstgenannte heisse Schmelze und kühlt auf 35 Celsiusgrade. Man gibt dann

0.5 GT Parfum dazu, verquirlt und kühlt auf Raumtemperatur.

Man erhält eine Nachtcrème mit überdurchschnittlicher Wirksamkeit.

## Ansprüche

1. Kosmetisches Präparat, mit einer wässrigen Phase, DADURCH GEKENNZEICHNET, dass es wenigstens flüssige und feste Bestandteile eines nach Champagnerart hergestellten Schaumweins in freier oder gebundener Form enthält.

2. Kosmetisches Präparat nach Anspruch 1, dadurch gekennzeichnet, dass die flüssigen Bestasndteile des Schaumweins zumindest den überwiegenden Teil der wässrigen Phase bilden.

3. Kosmetisches Präparat nach Anspruch 1 oder 2, dadurch gekennzeichnet, dass es die genannten Bestandteile eines Champagners enthält.

4. Kosmetisches Präparat nach Anspruch 3, dadurch gekennzeichnet, dass es die genannten Bestandteile eines zumindest vorwiegend aus Pinot-Noir-Trauben hergestellten Schaumweins, insbesondere Champagners enthält.

5. Kosmetisches Präparat nach einem der Ansprüche 1 bis 4, dadurch gekennzeichnet, dass es die genannten Bestandteile eines zumindest vorwiegend weissen Schaumweins, insbesondere Champagners enthält.

6. Kosmetisches Präparat nach den Ansprüchen 3 bis 5, dadurch gekennzeichnet, dass es die genannten Bestandteile eines zumindest vorwiegend Blanc-de-Noir-Schaumweins, insbesondere -Champagners enthält.

7. Kosmetisches Präparat nach einem der Ansprüche 1 bis 4, dadurch gekennzeichnet, dass es die genannten Bestandteile eines Rosé-Schaumweins, insbesondere -Champagners enthält.

8. Kosmetisches Präparat nach einem der Ansprüche 1 bis 7, dadurch gekennzeichnet, dass es Zellenextrakt enthält.